# EUROPEAN PATENT SPECIFICATION

(11) **EP 3 111 921 B1**
(45) Date of publication and mention of the grant of the patent: **29.04.2020**
(21) Application number: 16176299.2
(22) Date of filing: 27.06.2016
(51) Int. Cl.: A61K 8/894, A61K 8/02, A61K 8/86, A61Q 9/02, B26B 21/44, C10M 169/04

(54) **LUBRICATING MEMBER FOR RAZOR CARTRIDGES**
SCHMIERELEMENT FÜR RASIERKLINGENEINHEITEN
ÉLÉMENT DE LUBRIFICATION POUR CARTOUCHES DE RASOIR

(30) Priority: 30.06.2015 EP 15174448
(43) Date of publication of application: 04.01.2017
(73) Proprietor: The Gillette Company LLC, Boston, MA 02127 (US)
(72) Inventor: Bradford, Valerie Jean, Boston (US); Stephens, Alison Fiona, Egham, Surrey TW20 9NW (GB); Williamson, Martin Stephen, Egham, Surrey TW20 9NW (GB)
(74) Representative: P&G Patent Germany

(56) References cited:
- WO-A2-2007/031793
- GB-A- 1 299 089
- US-A1- 2014 323 374

## Description

### FIELD OF THE INVENTION

The invention relates to a lubricating member for razor cartridges comprising a silicone polyether bock copolymer exhibiting improved lubricating properties which can be readily manufactured without impacting performance.

### BACKGROUND OF THE INVENTION

The use of shaving aids in combination with razor blades to provide lubrication benefits during the shave is known, see for example US7,121,754, US6,298,558, US5,711,076, US5,134,775, US6,301,785, US 2009/0223057, US 2006/0225285, WO2007/031793, US 2014/0323374 and GB1299089. Such shaving aids typically comprise a water-insoluble matrix material to provide structural integrity and a water-soluble polymer, such as polyethylene oxide (polyox), in order to provide lubrication during the shave once the water-soluble polymer forms a solution with the water present during shaving. Since the introduction of polyox as a shaving lubricant, little development has been made in the field, even though polyethylene oxide polymers are not without limitations. For example, utilizing polyethylene oxide polymers having low molecular weights or high molecular weights may improve may provide a means to improve lubrication, but may also result in trade off with regard to residue and or stringiness or other aspects of the aqueous solution typically formed in-use. The resultant viscosity in aqueous solution may also increase, leading to negatively perceived attributes, for example concerning the feeling of the shave for the user, particularly in respect of the lubricant. The prior art does also describe the use of combinations of high and low molecular weight polyethylene oxide polymers in order to balance these performance attributes. Nevertheless, such combinations are also limited in their ability to improve performance and or suffer from other negative performance attributes. The art further describes the incorporation of additional materials to further improve the lubrication performance. For example US6442839, US2007/0110703 and US2009/0223057 describe the use of low levels of mineral and essential oils, butters, waxes and silicones. The use of mineral oil to enhance the glide performance is described in US2008/0060201. However the art also discloses a reduction of the swelling and solubility of the water soluble shaving aid contained in the water insoluble polymer matrix. The ability of the shaving aid to swell in contact with water is however believed to be the key mechanism by which the lubrication benefit is delivered to the skin. Hence this is not desirable, as it will negatively impact the overall performance.

Consequently, there is still a need to provide a lubricating member for razor cartridges comprising a water soluble polymer exhibiting improved lubricating properties which can be readily manufactured without impacting performance.

Silicone polyether block copolymers have been described in the literature to provide a number of benefits such as foaming, defoaming, wetting, deaeration and lubricity. However it has been now been surprisingly found that the selection of silicone polyether block copolymers having from 20% to 90% by weight of polypropylene, from 1% to 50% by weight of polyethylene oxide and from 1% to 20% by weight of silicone in combination with a water soluble polymer unexpectedly provide improved lubrication whilst ensuring the required level of water dispersion without impacting stringiness or residue. Moreover, the use of such silicone block copolymers may provide improved adhesion to the skin verses alternative materials such as copolymers of polyethylene oxide and polypropylene oxide

### SUMMARY OF THE INVENTION

One aspect of the invention relates to a lubricating member for use on a hair removal device, said lubricating member comprising a lubricating material comprising from 1 to 99% by weight of a water soluble polymer and from 0.1% to 70% by weight of a silicone polyether block copolymer, wherein said silicone polyether block copolymer comprises from 1 to 50%, by weight of polyethylene oxide, from 20% to 90% by weight of polypropylene oxide and from 1% to 20% by weight of silicone, wherein said silicone polyether block copolymer excludes additional alkyl chains.

### DETAILED DESCRIPTION OF THE INVENTION

### Lubricating material: Water soluble polymer

According to the present invention, the lubricating member comprises a lubricating material comprising from about 1% to about 99% by weight of water soluble polymer, preferably at least about 15%, more preferably at least about 20%, most preferably at least about 25%, and up to about 70%, preferably up to about 60% by weight of the lubricating member. The term water soluble polymer does not include the silicone polyether block copolymer as defined hereinafter.

Examples of suitable water soluble polymers include polyethylene oxide, polyvinyl pyrrolidone, polyacrylamide, polyhydroxymethacrylate, polyvinyl imidazoline, polyethylene glycol, polyvinyl alcohol, polyhydroxyethymethacrylate, copolymers of polyethylene oxide (PEO) and polypropylene oxide (PPO), guars, celluloses, modified celluloses and mixtures thereof. In some embodiments, said water soluble polymer is selected from high and or low molecular weight polyethylene oxides referred to in the industry as polyethylene oxide and polyethylene glycol respectively. Preferably the water soluble polymer is selected from the group consisting of polyethylene oxide, polyethylene glycol, copolymers of polyethylene oxide and polypropylene oxide and mixtures thereof.

The preferred water soluble polymers are the polyethylene oxides generally known as POLYOX (available from Union Carbide Corporation) or ALKOX (available from Meisei Chemical Works, Kyoto, Japan). The water soluble polymer, (especially these polyethylene oxides), may have average molecular weights of at least about 20,000, preferably at least about 50,000, more preferably at least about 100,000 or from about 100,000 to about 8 million, preferably about 300,000 to about 8 million, more preferably from about 1 million to about 5 million, even more preferably about 2 to 3 million. A particularly preferred polyethylene oxide comprises a blend of about 40% to 80% of polyethylene oxide having an average molecular weight of about 5 million (e.g. POLYOX COAGULANT) and about 60% to 20% of polyethylene oxide having an average molecular weight of about 300,000 (e.g. POLYOX WSR-N-750). The polyethylene oxide blend may also advantageously contain up to about 10% (for example about 5%) by weight of a low molecular weight (i.e. MW<10,000) polyethylene glycol such as PEG-100.

Suitable PEO/PPO copolymers may have an average molecular weight of at least 5,000, preferably in the range of from 10,000 to 20,000, more preferably from 11,000 to 15,000, even more preferably from 12,000 to 13,000 and even more preferably still from 12,250 to 12,750. Without wishing to be bound by theory, the inclusion of a PEO/PPO copolymer of sufficient molecular weight is thought to further improve the lubrication properties of the lubricating member in aqueous conditions, especially in combination with a further water soluble polymer (particularly polyethylene oxide), and thus prevent an undesirable feeling in use.

The PEO/PPO copolymer may advantageously be a block copolymer, preferably a tri-block copolymer having the sequence: PEO-PPO-PEO, the later commercially available under tradenames such as Pluracare from BASF and Pluronic from Sigma-Aldrich.

The PEO/PPO copolymer may have a weight ratio of PEO to PPO (i.e. of ethylene oxide repeat units to propylene oxide repeat units), of from 1000:1 to 1:1000 or from 100:1 to 1:100. The PEO/PPO copolymer is typically present at an amount of from 0.01% to 50%, preferably from 0.01% to 50%, more preferably from 2% to 40%, even more preferably from 3% to 25%, even more preferably still from 4% to 20% and most preferably from 5% to 10% by weight of the lubricating material or by weight of the lubricating member.
The lubricating member and or water soluble polymer preferably comprises less than 5%, preferably less than 1% by weight and more preferably is/are substantially free of lathering soaps (i.e. salts of fatty C4 to C30 acids) and lathering surfactants. A lathering surfactant is defined as a surfactant which when combined with water and mechanically agitated, generate a foam or lather. Lathering surfactants include anionic and amphoteric lathering surfactants and mixtures thereof. Anionic lathering surfactants include sarcosinates, sulfates, sulfonate, isethionate, taurates, phosphates, lactylates, glutamates, alkali metal salts of fatty acids (i.e. soaps) having from 8 to 24 carbons, and mixtures thereof.

### Lubricating material: Silicone polyether copolymer

According to the invention, the lubricating material further comprises from about 0.1% to about 70%, preferably from about 1% to about 20%, more preferably from about 1% to 15%, even more preferably from about 1% to about 5% or alternatively from about 40% to about 60%, more preferably from about 45% to about 55%, or alternatively from about 10% to about 60%, preferably from about 20% to about 40% by weight of a silicone polyether copolymer or mixtures thereof.

The silicone polyether copolymer comprises from about 1% to 50%, by weight of polyethylene oxide, from about 20% to about 90% by weight of polypropylene oxide and from about 1% to about 20% by weight of silicone. Preferably the silicone polyether copolymer comprises at least about 40%, more preferably at least about 50%, most preferably at least about 60% by weight of polypropylene oxide. In addition, the silicone polyether copolymer preferably comprises at least about 10%, more preferably from at least about 15%, most preferably from about 15% to 30% by weight of polyethylene oxide. Furthermore, the silicone polyether block copolymer comprises from 1%to 20%, preferably 10% to 20%, more preferably about 15% by weight of silicone.

Whilst silicone polyether block copolymers are known in the art to provide a number of benefits such as foaming, defoaming, wetting, deaeration and lubricity, it has been now been surprisingly found that the selection of silicone block copolymers having from 20% to 90% by weight of polypropylene oxide and from **1**% to 50% of polyethylene oxide unexpectedly provide improved lubrication whilst ensuring the required level of water dispersion and or solubility verses silicone polyether block copolymers having less or no polypropylene oxide and more polyethylene oxide. Moreover, the use of such silicone block copolymers provides improved adhesion to the skin verses alternative materials such as copolymers of polyethylene oxide and polypropylene oxide.
Furthermore, the inclusion of **1**% to 20% of silicone by weight of the silicone polyether block copolymer surprisingly provides desirable levels of lubrication despite being present at low levels in the polymer.

The copolymers are block copolymers and may preferably have a pendant graft structure. The silicone polyether block copolymer comprises from **1**% to 50%, preferably from 10% to 20%, more preferably about 20% by weight of polyethylene oxide. The silicone polyether block copolymer comprises from 20% to 90%, preferably from 40% to 90%, more preferably from 50% to 80%, most preferably about 65% by weight of polypropylene oxide. The silicone polyether block copolymer comprises from **1**% to 20%, preferably 10% to 20%, more preferably
about 15% by weight of silicone.

The silicone polyether block copolymer preferably has a ratio of polyethylene oxide units to polypropylene oxide units of from 2.0 to 0.1, more preferably from
0.6 to 0.25. The silicone polyether block copolymer preferably has a ratio of polyethylene oxide units to polypropylene oxide units to silicone units of from20:65:15.

The silicone polyether copolymer may have a molecular weight of from about 10000 to about 19000, more preferably from about 10000 to 15000.

In a preferred embodiment the silicone polyether copolymers suitable for use herein only contain repeating units of silicone, polyethylene oxide and polypropylene oxide. Silicone polyether copolymers comprising additional alkyl chains are excluded.

Suitable silicone polyether copolymers are available from Momentive under the Silwets trademark products including L7210. Preferably the silicone polyether block copolymer is liquid at 25°C, so that it can be provided in a liquid form for spray coating manufacturing methods. The melting point is determined according to ASTM D5440-93. Preferably the silicone polyether block copolymer is sparingly soluble, preferably soluble or more preferably freely soluble in water according to the United States' Pharmacopeia (USP) definition in 31/NF 26 Vol. 2 General Notices, Page Xvii. According to that definition, sparingly soluble means 30 to 1000 parts of water are needed to dissolve 1 part solute, soluble means that 10 to 30 parts of water are needed to dissolve 1 part solute and freely soluble means than from 1 to 10 parts of water are needed to dissolve 1 part of solute.

In one embodiment the lubricating member comprises silicone polyether block copolymer and a water soluble polymer, preferably polyethylene oxide at a weight ratio of from 1:8 to 8:1, preferably from 1:5 to 5:1, more preferably from 1:3 to 3:1 and even more preferably from 1:2 to 2:1.

### Lubricating material: Optional Hydrophobic compound

According to the invention the lubricating member may further comprise a hydrophobic compound or mixtures thereof. In one embodiment the lubricating member comprises from 1% to 40%, preferably from 5% to 40%, more preferably from about 10% to about 40%, even preferably from about 12% to about 30% by weight of a hydrophobic compound and or mixtures thereof. Suitable hydrophobic compounds include natural oils and or waxes and or fats; synthetic waxes or oils; triglycerides; skin active agents, sensates, fragrance oils, silicones and mixtures thereof. The hydrophobic material can provide a number of in use benefits such as lubrication, skin feel and cooling sensation.

The hydrophobic compound may comprise skin active agents such as, but not limited to oil soluble vitamins, such as vitamin E derivatives, including vitamin E acetate and tocopherol nicotinate; oil-soluble vitamin A derivatives, such as retinyl palmitate; lanolin; ceramides; sterols and sterol esters; salicylic acid; camphor; eucalyptol; essential oils; peppermint oil, Iso E Super [(1-(1,2,3,4,5,6,7,8-octahydro-2,3,8,8-tetramethyl-2-naphthalenyl)ethanone]; and mixtures thereof.

In some embodiments, the hydrophobic compound comprises one or more sensates. A large number of coolant compounds of natural or synthetic origin are known. The most well know is peppermint oil. Among synthetic coolants, many are derivatives of or are structurally related to menthol, i.e., containing the cyclohexane moiety, and derivatized with functional groups including carboxamide, ketal, ester, ether and alcohol. Non-limiting examples include methyl emthylamido oxalate, (under the tradename Frescolat X-cool available from Symrise), menthyl lactate (such as Frescolate ML Natural available from Symrise), and Menthyl Pyrrolidone Carboxylate also known as Menthyl PCA (under the tradename Questices available from Givaudan).

Hydrophobic compounds may be selected from capric and or caprylic triglycerides, grape seed oil, olive oil, micro- crystalline wax, shea butter, cocoa butter, lanolin, essential oil, peppermint oil, isohexadecane, petrolatum, silicone polymers including waxes and oils (selected from dimethicones, phenylated silicones and mixtures thereof) and mixtures thereof.

### Optional ingredients: Hydrophobic binders

The lubricating member may also further comprise a water-insoluble material such as hydrophobic binders. Such components may enhance the life of the lubricating material by reducing its tendency to be mechanically eroded. Advantageously, the hydrophobic binder is solid at standard temperature and pressure. Suitable hydrophobic binders include divalent metal cation stearate, preferably magnesium stearate, calcium stearate, zinc stearate, or mixtures thereof, more preferably magnesium separate; ethyl cellulose; polycaprolactone; polyethylene; polypropylene; polystyrene; butadiene-styrene copolymer (e.g. medium and high impact polystyrene); polyacetal; acrylonitrilebutadiene-styrene copolymer; ethylene vinyl acetate copolymer and blends such as polypropylene/polystyrene blend; and mixtures thereof. Preferred water insoluble materials are polyethylene, polypropylene, polystyrene; butadiene-styrene copolymer including medium and high impact polystyrene, polyacetal, acrylonitrilebutadiene-styrene copolymer, ethylene vinyl acetate copolymer and mixtures thereof. The lubricating material may comprise from about 1 to about 50%, preferably from about 10% to about 40% more preferably from about 20% to about 40% by weight of hydrophobic binder. The hydrophobic binder may fall under the definition of hydrophobic compound as used herein and in such a case should be included for purposes of determining the amount by weight of the hydrophobic compound or mixture.

In some embodiments, the lubricating material may comprise any other ingredients commonly found in commercially available shaving aid members. The lubricating member may therefore contain other conventional shaving aid ingredients, such as low molecular weight water-soluble release enhancing agents such as polyethylene glycol (MW<I0,000, e.g., 1-10% by weight PEG-100), water-swellable release enhancing agents such as cross-linked polyacrylics (e.g., 2-7% by weight), colorants, skin feel / care actives such as water soluble cationic polymers, surfactants, soaps (including interrupted soaps), antioxidants, preservatives, emollients, beard softeners, astringents, medicinal agents, plasticizers, additional lubricants, depilatories/keratolytic materials, tackifiers, skin-soothing agents, fragrances, compatibilisers, anti-inflammatory agents, antipruritic/counterirritant materials etc and mixture thereof. These ingredients may fall under the definition of hydrophobic compounds as used herein and should be included as such in determining the amount of hydrophobic compounds.

The lubricating member comprising the combination of water soluble polymer and silicone polyether block copolymer as defined in claim 1 and preferably comprising any optional components present, may have a coefficient of friction as defined according to the method described herein below of 0.0300 or less, preferably of 0.0275 or less, more preferably of 0.0250 or less in order to improve lubrication.

### Method of manufacture/processing

The lubricating member may be formed using any method known in the art such injection molding, pressing, impregnation, spray-coating, calendaring and extrusion. All of the components of the lubricating member can be blended prior to molding or extrusion. For best results, it is preferred that the components are dry. In summary, the method comprises the steps of providing a feed comprising the water soluble polymer and silicone polyether block copolymer, and molding, pressing, impregnating, spray-coating, calendaring and/or extruding said feed to form a solid lubricating member. Additional optional steps may be included depending on the process of manufacture which is utilized for example heating said feed to a temperature of from about 120°C to about 200°C.

For example, the blended components may be extruded through a Haake System 90, ¾ inch (∼1.91 cm) diameter extruder with a barrel pressure of about 1000-2000 psi (∼6.90 - 13.8 MPa), a rotor speed of about 10 to 50 rpm, and a temperature of about 150°-185°C and a die temperature of about 170°-185°C. Alternatively, a 1¼ inch (∼3.18 cm) single screw extruder may be employed with a processing temperature of 175°-200°C, preferably 185°-190°C, a screw speed of 20 to 50 rpm, preferably 25 to 35 rpm, and an extrusion pressure of 1800 to 5000 psi (∼12.4 - 34.5 MPa), preferably 2000 to 3500 psi (∼13.8 - 24.1 MPa). The extruded member is air cooled to about 25°C. To injection mold the lubricating member it is preferred to first extrude the powder blend into pellets. This can be done on a 1¼ or 1½ inch (∼3.18 or 3.81 cm) single screw
extruder at a temperature of 120°-180°C, preferably 140°-150°C, with a screw speed of 20 to 100 rpm, preferably 45 to 70 rpm. The pellets are then molded in either a single material molding or multi-material molding machine, which may be single cavity or multi-cavity, optionally equipped with a hot-runner system. The process temperature can be from 165° to 250°C, preferably from 180° to 225°C. The injection pressure should be sufficient to fill the part completely without flashing. Depending on the cavity size, configuration and quantity, the injection pressure can range from 300 to 2500 psi (∼2.07 - 17.2 MPa). The cycle time is dependent on the same parameters and can range from 3 to 30 seconds, with the optimum generally being about 6 to 15 seconds.

The lubricating member may be manufactured using a hot melt process. In such processes the waxes are melted in a water bath to a temperature of 85°C stirred until completely melted. The liquid silicone polyether block copolymer is then added and stirred. The temperature is then reduced to about 55°C when the remaining components are added whilst stirring. The molten material is then poured into a mold and pressure applied. The member is removed from the mold upon cooling.

In another embodiment, the lubricating member may alternatively be provided in the form of a compressed powder. For such embodiments, the lubricating member may be manufactured whereby the water soluble polymer and other solid dry components are provided as particulates and mixed. The particulate material(s) is solid at 25°C and preferably has a melting point of 30°C or more. The lubricating member thus may comprise from 10% to 90% by weight of a particulate material(s) of a water soluble polymer or mixtures thereof.

The silicone polyether block copolymer is, if necessary, liquefied and then spray coated onto at least a portion of the water soluble polymer particulate and other dry components if present. Preferably at least 95%, more preferably at least 98% and even more preferably substantially 100% of the water soluble polymer and preferably any other dry particulate material components present are spray coated with the silicone polyether block copolymer. Hydrophobic compounds if present may also be spray coated as a mixture with the silicone polyether block copolymer or in a separate spray coating step. The resulting mixture is then compression compacted to form a tablet.

The term compression and or compression molding or compression compaction as used herein refers to a process by which the bulk density of a particulate or powder is reduced to form a solid tablet by the application of pressure. Typically, this is performed without the application of external shear force or heat. Preferably the compression compaction is conducted below the melting point of at least one, preferably all the particulate components, preferably at ambient temperature of 25°C. As such the particulates retain their integrity after the compression process and are typically visible by the naked eye after the compression process is completed.

In certain embodiments, additional energy sources such as heat may be applied during or post compression to increase inter-particulate bonding and increase the rigidity of the resulting lubricating member but which preferably does not resulting in any substantial melting of the particulate material. Preferably however this method does not require an extrusion or injection molding step or the application of energy sources such as heat.

The lubricating member may thus be provided in the form of a compressed powder from particulates. Preferably the particulates have an average particle size distribution of from about 50 to 1250 microns and preferably from about 300 to 1250 microns, more preferably about 1000 microns. Alternatively the particulate size is such that 90% of particles pass through a 20 mesh screen; i.e. 90% of particles are less than 841micron in diameter. The lubricating member is compressed preferably directly into a preform or container with a compression force of typically greater than 1KN. This may be achieved using any method and equipment known in the art in the art such as a die press. The bulk density of the particulate material prior to compression is typically about 300 to 600 kg/m³ and increases to about 1000 to 1200kg/m³ following compression. Bulk density thus may be increased by about 200% to about 400% after the compression. Whilst no bound by theory, it has been found that the use of particulate compression manufacturing, preferably cold particulate compression (i.e. at 25°C or less) to form the lubricating member enables highly lubricous components to be incorporated therein whilst not negatively impacting the water solubility and swelling performance of the water soluble polymer. This also allows flexibility in the size of the resulting lubricating member to be used for multiple razor cartridges whilst enabling the effective delivery of the silicone polymer to the skin of the consumer in use.

### Hair Removal Head

According to some embodiments of the invention, the lubricating member finds particular application for hair removal devices. Hair removal devices generally comprise a hair removal head and a handle or grip portion, upon which the hair removal head is mounted. The hair removal device can be manual or power driven and can be used for wet and/or dry application. The hair removal head can include a wide scraping surface such as where the hair removal device is used with a depilatory, or be a razor cartridge or foil where the device is a shaving razor. The hair removal head may be replaceable and/or pivotally connected to a cartridge connecting structure and in turn or independently (e.g. permanently fixed) to a handle. In some embodiments, the cartridge connecting structure includes at least one arm to releasably engage the hair removal head.

The hair removal head typically comprises one or more elongated edges usually positioned between a first and second end, said one or more elongated edges comprising a tip extending towards said first end. Where the hair removal head is a razor cartridge the one or more elongated edges can include blades. For example, U.S. Patent 7,168,173 generally describes a Fusion® razor that is commercially available from The Gillette Company and which includes a razor cartridge with multiple blades. Additionally, the razor cartridge may include a guard as well as a skin engaging member. A variety of razor cartridges can be used in accordance with the present invention. Non limiting examples of suitable razor cartridges, with and without fins, guards, and/or shave aids, include those marketed by The Gillette Company under the Fusion®, Venus® product lines as well as those disclosed in U.S. Patent Nos. 7,197,825, 6,449,849, 6,442,839, 6,301,785, 6,298,558; 6,161,288, and U.S. Patent Publ. 2008/060201. Those of skill in the art will understand that the lubricating member can be used with any currently marketed system or disposable razor, including those having 2, 3, 4 or 5 blades. In such a case, the hair removal device is a razor, the hair removal head is a razor cartridge and the one or more elongated edges are blades. Another example of a hair removal device is a scraping tool for use with a hair removal composition, i.e. a depilatory.

In some embodiments, said at least one lubricating member is located on the portion of the cartridge that contacts skin during the hair removal process, forward and/or aft of the blades. A feature "forward" of the one or more elongated edges, for example, is positioned so that the surface to be treated with by the hair removal device encounters the feature before it encounters the elongated edges. A feature "aft" of the elongated edge is positioned so that the surface to be treated by the hair removal device encounters the feature after it encounters the elongated edges. Where more than one lubricating member is provided on the hair removal device, they can be the same (identical) or different, in terms of physical shape/structure and/or chemical composition, and one or more of them may comprise the spray coated particulate.

In some particular embodiments, a plurality (e.g. 2, a first and second) of lubricating members may be provided on the hair removal head, with the first skin engaging member comprising the same composition or different. These lubricating members may be placed collectively (for example adjacent to one another) ahead of or behind the elongated edges (e.g. blades on a razor cartridge), including side by side, or separately with one ahead of the elongated edges and the other behind.

The lubricating member may be free standing utilizing a suitable attachment means such as adhesive or may be contained at least partially within a container.

The container typically has a base and at least one side wall extending vertically preferably perpendicular from said base and a skin contacting surface. In a preferred embodiment said container comprises a base and at least 2 side walls, more preferably at least 4 side walls, preferably said walls completely enclosing the base. Typically, each pair of walls are substantially parallel and preferably one pair of walls is substantially parallel to the at least two blades. Alternatively, the base may be enclosed by a one piece single wall. The container may form any shape including substantially rectangular, or oval. The container typically has a front wall adjacent the blades and a rear wall, preferably substantially parallel thereto and furthest from said blades.

The container is preferably further provided with at least one dispensing orifice for dispensing the lubricating member onto the skin during use. In one embodiment the container is provided with a top extending substantially perpendicular from the side wall (s). The container would in such an embodiment typically have a receiving region for receiving the lubricating member. The top may be substantially parallel to the base or it may be provide at an angle such that the distance of the top from the blade plane increases or decreases as the distance of the container from the blades increases. In one embodiment the height of the top of the container increases in distance from the blade plane as the container distance from the blades increases. In an alternative embodiment the height of the top of the container decreases in distance from the blade plane as the container distance from the blade increases.

The orifice may be of any shape and may, for example, have a cross sectional area of from about 0.00324 to about 1.613 cm². Small orifices can also be provided with cross sectional area of from about 0.0324 to about 0.324 cm², or from about 0.0645 to about 0.16135 cm². Larger orifices can have cross sectional areas of from about 0.324 to about 1.613 cm², or from about 0.645 to about 1.29 cm². The container may comprise a single orifice or multiple orifices which may be large and or small. In one embodiment the container comprises at least two orifices. Combinations of small and large orifices can also be provided on the same skin engaging member, or on separate members on the same cartridge, depending on the desired dispense rate and amount of exposure of the lubricating material to water. In one embodiment the top of the container is provide with one preferably two orifices, more preferably two substantially identical orifices adjacent one another.

The skin engaging surface of the container which has a surface area, while the at least one orifice (i.e. the sum for all orifices if a plurality are present) has a cross sectional area such that the surface area and cross sectional area are in a ratio of from about 50:1 to about 1:1, or about 25:1 to about 2:1, or about 10:1 to about 3:1.

In some embodiments, at least a portion of said container is not linear for example angled or curvilinear. Curvilinear as defined herein means that at least a portion is curved such that it does not form a straight line. Where at least two containers are provided, they can also be positioned relative to one another such that they do not form a straight line. Alternatively, the curved or angled nature is such that it forms at least a partial ring. A partial ring, as defined herein, means that the structure has at least two curved or angled sections which are concave to form an inner region. The partial ring can also include a curved or angled portion which is positioned convex to said inner region. One or more of said containers may also be positioned relative to one another to form a full ring.

The container can be formed of a variety of materials. The container may, preferably be for example, provided from a non-water soluble material such that it does not degrade or dissolve during normal use.

The container typically has sufficient mechanical strength and rigidity to provide adequate mechanical strength to the entire skin engaging member, both as initially produced and after a significant amount of lubricating material has leached out of the container. Alternatively or in addition a further reinforcing member may also be utilized. In some embodiments, the container comprises a base and one or more side walls, forming a receiving region, or channel, onto or into which the lubricating material is placed.

The side walls may or may not be the same height (as measured extending away from the base of the container). At least one of the side walls can have a height of about 0.1 cm to about 1 cm, preferably from about 0.2 cm to about 0.4 cm. The pair of side walls can be biased away from each other as the walls extend away from said base, or they can be biased towards each other. At least one wall extends vertically from the base and is preferably perpendicular to the blade plane (P). One or both ends of the container can be enclosed, e.g. as described in US 7,581,318. The term maximum height of at least one wall as used herein refers to the first front wall preferably substantially parallel to the at least two blades and closest thereto or it refers to the rear wall farthest from said at least two blades. In one embodiment the said at least one wall is closest to said at least two blades. In an alternative embodiment the at least one wall is farthest from said at least two walls. In one embodiment, the ratio of the height of the front wall to the rear wall is from 5:1 to 1:5, more preferably from 2:1 to 1:2 and more preferably the height of the front wall is greater than the rear wall. The walls have a thickness of from 0.1cm to 1.0cm, preferably from 0.3 to 0.5cm.

The container may be made of a water-insoluble polymer, particularly a thermoplastic resin. Thermoplastic resins are those materials which can be extruded or molded into a shape and are resilient under normal environmental conditions such as contact with water, even up to normal household hot water temperatures (for example up to 125 °C); normal wear and tear by consumers during use; device assembly and shipping, etc. Thermoplastic resins suitable for use in the carrier include polystyrene, high impact polystyrene (polystyrene-butadiene), polypropylene, filled polypropylene, polyethylene, nylon ethylene vinyl acetate, and blends such as 70% nylon/30% polyethylene oxide, 60% polystyrene/40% polyethylene oxide butadiene styrene copolymer, polyacetal, acrylonitrile-butadiene styrene copolymer, and mixtures thereof. The preferred resins are high impact polystyrene, polystyrene, ethylene vinyl acetate (EVA), and mixtures thereof.

In some embodiments, the cartridge comprises a guard comprising at least one elongated flexible protrusion to engage a user's skin. The at least one flexible protrusion may comprise flexible fins generally parallel to said one or more elongated edges. Said at least one flexible protrusion may additionally or alternatively comprise flexible fins comprising at least one portion which is not generally parallel to said one or more elongated edges. Non-limiting examples of suitable guards include those used in current razor blades and include those disclosed in U.S. Patent Nos. 7,607,230 and 7,024,776; (disclosing elastomeric / flexible fin bars); 2008/0034590 (disclosing curved guard fins); 2009/0049695A1 (disclosing an elastomeric guard having guard forming at least one passage extending between an upper surface and a lower surface). In some embodiments, said lubricating member is positioned on the cartridge aft of the guard and forward of said elongated edge. In another embodiment, the lubricating member is positioned on the cartridge forward of the guard. This embodiment can be particularly useful to deliver the lubricating member prior to contact with the guard.

### Exemplified compositions:

### I. Examples

The following examples formulations of the lubricating members of the invention were made according to the tables below by dissolving the listed ingredients into water using gentle agitation with an overhead stirrer until a solution was formed in order to determine the coefficient of friction thereof.

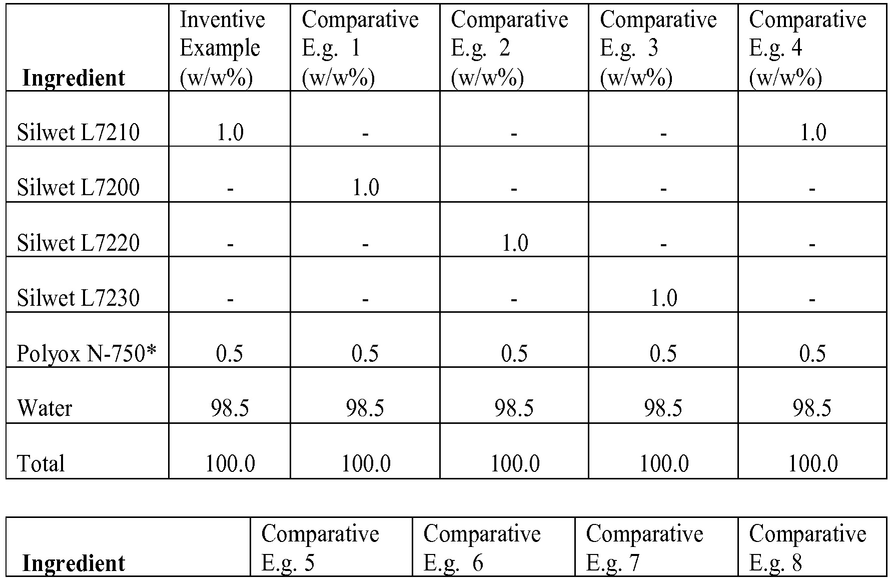

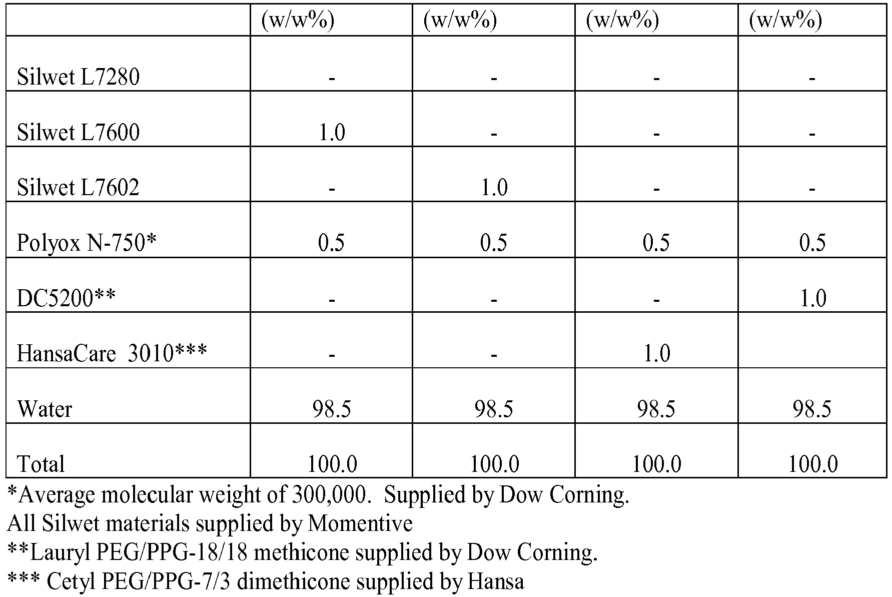

### Silicone polyether block copolymer composition from Momentive supplier information

| **Ingredient** | **Silicone % w/w*** | **PPO % w/w*** | **PEO %w/w*** |
|---|---|---|---|
| Silwet L7210 | 15 | 65 | 20 |
| Silwet L7200 | 34 | 22 | 44 |
| Silwet L7220 | 25 | 55 | 20 |
| Silwet L7230 | 24 | 44 | 30 |
| Silwet L7280 | 38 | 33 | 29 |
| Silwet L7600 | 25 | 0 | 75 |
| Silwet L7602 | 60 | 0 | 40 |

The coefficient of friction (COF) of each of the above solutions was measured using a industry standard rotational tribometer, MTM2 machine from PCS Instruments. In the event that any of the components are not fully soluble, these components are separated from the solute prior to determining the COF. The COF is determined only on the solute. The device has a specimen disc mounted on a vertical shaft within a stainless steel test fluid reservoir. A ball is end mounted onto a pivoting shaft and is automatically loaded against the specimen disc at the start of the test. The test fluid is filled into the reservoir. DC servomotors rotate the disk with accurate speed and the traction force applied to the ball specimen is measured with a high sensitivity force transducer, with additional sensors to measure applied load, lubricant temperature and the electrical contact resistance. From this data the coefficient of friction is calculated.

For the above fluids the following conditions were used;
Ball: ¾ inch stainless steel ball (supplied by PCS Instruments)
Specimen disc: 46mm diameter PDMS elastomer disc (material supplied by PCS Instruments)
Test Load: 5N
Test Speed: 150 mm/s (shave relevant speed)
Temperature: 25°
Specimen fluid: 40 ml

### I Results:

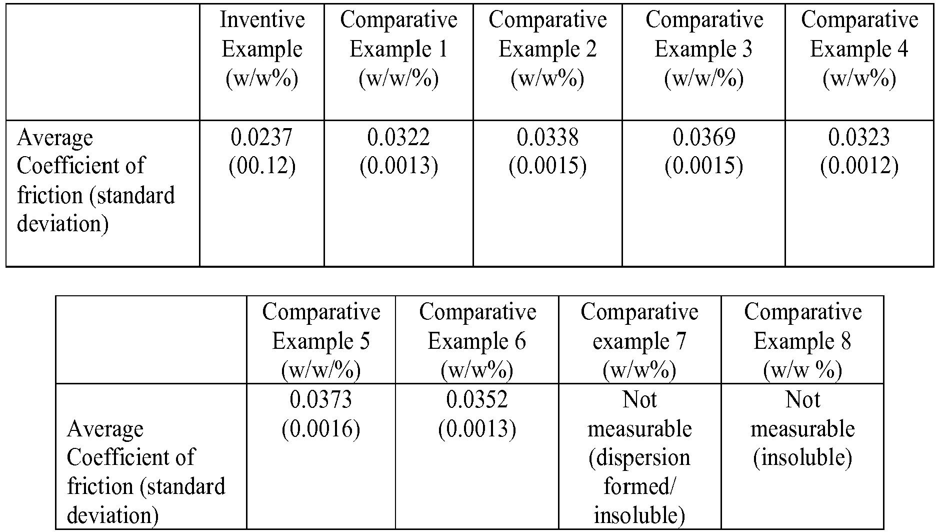

The data clearly shows that the solutions of silicone polyether copolymer having a low level of silicone and polyethylene and a high level of polypropylene oxide (Inventive example 1) in combination with a water soluble polymer as claimed significantly reduces the coefficient of friction verses polymers with higher levels of silicone and or low levels of polypropylene oxide an higher levels of polyethylene oxide in combination with a water soluble polymer (comparative examples 1-6). Comparative examples 7 and 8 comprise polymers which contain lauryl alkyl chains. These examples did not dissolve to form a solution and thus the coefficient of friction could not be determined.

### II. Examples

The following examples were made according to the table below by dissolving the listed ingredients into water using gentle agitation with an overhead stirrer until a solution was formed. A 15 legged experiment was designed to evaluate the above three variables. Silwet 7210 was used in all the examples. The remainder of the formula was DI water. Each of the 15 legs was tested on the MTM2 tribometer using a disc speed range 500 - 1 mm s⁻¹ and a new PDMS disc for each sample. Each solution was repeated four times and a mean COF (and standard deviation) was calculated from the speed region of 90-200mms.

| | **Polyox N750 0.3MM % w/w** | **Polyox N12k 1.0MM %w/w** | **Polyox N60K 2.0MM % w/w** | **Polyox 301 4.0MM % w/w** | **Silwet % w/w** | **Avg COF (sd)** |
|---|---|---|---|---|---|---|
| 1 | 0.2 | - | - | - | 0.07 | 0.0458 (0.0029) |
| 2 | 1.0 | - | - | - | 0.2 | 0.0416 (0.0025) |
| 3 | 1.0 | - | - | - | 0.5 | 0.0396 (0.0018) |
| 4 | - | 0.2 | - | - | 0.04 | 0.0409 (0.0029) |
| 5 | - | 0.6 | - | - | 0.3 | 0.0335 (0.0012) |
| 6 | - | 1.0 | - | - | 0.2 | 0.0325 (0.0011) |
| 7 | - | - | 0.2 | - | 0.04 | 0.0359 (0.0011) |
| 8 | - | - | 0.2 | - | 03 | 0.0322 (0.0009) |
| 9 | - | - | 0.6 | - | 0.2 | 0.0302 (0.0009) |
| 10 | - | - | 1.0 | - | 0.2 | 0.0315 (0.0013) |
| 11 | - | - | 1.0 | - | 0.5 | 0.0287 (0.0010) |
| 12 | - | - | - | 0.2 | 0.07 | 0.0335 (0.0012) |
| 13 | - | - | - | 0.6 | 0.12 | 0.0311 (0.0011) |
| 14 | - | - | - | 0.6 | 0.3 | 0.0343 (0.0016) |
| 15 | - | - | - | 1.0 | 0.33 | 0.0301 (0.0015) |

The above results were then modeled in order identify a preferred formula to reduce the coefficient of friction. Sensory testing was then performed on this preferred formula versus the corresponding non-silicone polyether copolymer containing control to demonstrate the performance improvement.

Sensory testing was conducted upon a naive panel (N=5) with 3-overlapping strokes being performed on their forearms. The order in which each panelist received the products and the first forearms used were randomized. The procedure used was as follows;
1. Wash both forearms thoroughly with warm water and soap to remove any oils or moisturizers that may already be on the skin.
2. Mark out a 15cm x 5cm area on the inner forearm using the template provided and evenly apply 0.1mL amount of fluid.
3. After 3 overlapping strokes using plastic plaque of size (12mm x 40mm) and immediately rate:
   a. Not (0) - Very Glidey (10)
   b. Draggy (0) - Very lubricated (10)
4. Using index finger on forearm rate:
   c. Not Smooth (0) - Extremely Smooth (10)
5. Rinse fore-arm, pat dry and wait for 2mins. Then re-rate smooth attribute as above.

| Property (delta score vs control) | Glide | Lubricated | Smooth (during & after use) |
|---|---|---|---|
| Test Formula: 0.64% w/w 2MM Polyox N60K + 0.24% w/w Silwet L7210 | 0.8 | 0.6 | 0.4 and 0.6 |
| Control: 0.64% w/w 2MM N60K Polyox | | | |

The sensory data indicates a consumer relevant measurable improvement on improved glide, less drag and a smooth feel during and after use verses a control comprising only 0.64% polyethylene oxide (2MM Polyox).

### III. Formulation Examples

| **Ingredient** | **Example 1 (% w/w)** | **Example 2 (%w/w)** | **Example 3 (%w/w)** |
|---|---|---|---|
| Polyox WSR coag | - | 10 | 20 |
| Polyox N60k | 30 | - | - |
| Silwet L7210 * | 20 | 50 | 20 |
| Softcat SL5 ** | - | - | 10 |
| Nhance 3196 *** | | 10 | - |
| Petrolatum | - | - | 20 |
| DC200, 350cst $ | 20 | - | - |
| Cetyl alcohol | 30 | 25 | 25 |
| Multiwax 180MH # | - | 5 | 5 |
| Total | 100 | 100 | 100 |

| | | | |
|---|---|---|---|
| Suppliers: * Momentive,** Dow Chemicals, *** Ashland, $ Dow Corning, # Sonnenborn | | | |

Formulation Examples 1 -3 were prepared as follows:
1. Sanitize all equipment
2. Turn on water bath/ vessel jacket to 85° C
3. Add waxy phase ingredients (cetyl alcohol, multiwax 180MH) and stir with overhead stirrer until completely melted
4. Add oil phase ingredients (petrolatum, DC200, Silwet L7210) and mix until fully liquid
5. Reduce heat to 55° C and add powder ingredients (polyox (WSR or N60K), Nhance 3196 and Softcat SL5) until fully dispersed.
6. Pour mixture into a mould
7. Assemble part onto razor cartridge.

### IV. Formulation Examples

| Ingredients | **Example 4 (% w/w)** | **Example 5 (%w/w)** |
|---|---|---|
| Polyox WSR coag | 98.0 | - |
| Polyox N60k | | 88.0 |
| Silwet L7210 | 2.0 | 2.0 |
| Pluronic F127 * | - | 10.0 |
| Total | 100 | 100 |

| | | |
|---|---|---|
| Suppliers: * BASF | | |

The lubricating members were prepared by dry mixing the dry ingredients in the table above and then spray coating the silwet L7210 onto the powder blend. An appropriate amount of the resulting mix was then compressed and compacted into a suitable container for attachment to a razor cartridge, using a die press at 2.2KN for about 5 seconds.

### V. Formulation Examples

| Ingredient | **Ex. 6 (% (w/w)** | **Ex. 7 (% w/w)** |
|---|---|---|
| Sodium Stearate | 25.4 | 26.2 |
| Propylene Glycol | 14.2 | 13.5 |
| Glycerin | 12.4 | 12.4 |
| Aqua | 12.0 | 12.0 |
| Sorbitol | 6.8 | 6.8 |
| Sodium Laureth Sulfate | 6.8 | 6.8 |
| Sodium Myristate | 4.8 | 4.8 |
| Squalane | 2.2 | 2.2 |
| Olea Europaea (Olive) Fruit Oil | 1.6 | 2.0 |
| Persea Gratissima (Avocado) Oil | 1.6 | 2.0 |
| Paraffinum Liquidum/Huile Minerale | 1.3 | 1.3 |
| Polyethylene * | 1.3 | 1.3 |
| Polybutene ** | 1.3 | 1.3 |
| PEG-90M | 1.2 | 1.2 |
| PEG-45M | 1.2 | 1.2 |
| Silwet L7210 | 1.0 | 1.0 |
| Lauryl Dimethicone /Polyglycerin-3 Crosspolymer *** | 1.1 | 1.1 |
| Garcinia Indica Seed Butter | 0.8 | 1.0 |
| Parfum | 1.0 | 1.0 |
| PEG-7M | 0.6 | 0.6 |
| Stearic acid | 0.4 | 0.3 |
| Total | 100.0 | 100.0 |

| | | |
|---|---|---|
| : * Suppliers: *Sensient, **Shin-Etsu | | |

The above ingredients for examples 6 and 7 including the silicone polyether block copolymer (Silwet) can be formed into a lubricating member according to the methods of making the molded shaving aid composition described in US7811553 paragraphs [0059-0081], in either the poured soap base or process sensitive phase, as well as within a one-step batch process, or a continuous process. It is preferred to incorporate the polyethylene glycols into the process sensitive phase to minimize degradation.

### VI. Formulation Examples

| Ingredient | **Ex.1 (% w/w)** | **Ex. 2 (%w/w)** | **Ex.3 (%w/w)** |
|---|---|---|---|
| HIPS Ineos 5410 | 26.50 | - | 30.00 |
| Elvax 660 | - | 26.50 | - |
| Polyox WSR Coagulant | 33.56 | 33.56 | 38.75 |
| Polyox N750 | 22.69 | 22.69 | 25.00 |
| PCL CAPA 6506 | 5.00 | 5.00 | - |
| Colourant | 4.00 | 4.00 | 1.00 |
| Irganox Antiox B-215 | 0.25 | 0.25 | 0.25 |
| Silwet L-7210 | 3.00 | 3.00 | 3.00 |
| PEG 4600 | 5.00 | 5.00 | 5.00 |

### Example 1:

The lubricating members were prepared by dry mixing the dry ingredients in the table above and then spray coating the silwet L7210 onto the powder blend. The blended components maybe extruded through a Rondol 18, 18mm diameter extruder with a barrel pressure of about 1000-2000 psi (∼6.90 - 13.8 MPa), a rotor speed of about 10 to 50 rpm, and a temperature of about 150°C-185°C and a die temperature of about 170°C -185°C. Alternatively, a 1 1/2 inch (∼3.81 cm) single screw extruder may be employed with a processing temperature of 175 °-200 ° C, preferably 185°C -190°C, a 30 screw speed of 20 to 50 rpm, preferably 25 to 35 rpm, and an extrusion pressure of 1800 to 5000 psi (∼12.4 - 34.5 MPa), preferably 2000 to 3500 psi (∼13.8 - 24.1 MPa). The extruded strip is air cooledto about 25°C. To injection moldthe strips it is preferred to first extrude the powder blend into pellets. This can be done on a 1 1/4 or 1 1/2 inch (∼3.18 - 3.81 cm) single screw extruder at a temperature of 120°C -180 °C, preferably 140°C -150° C, with a screw speed of 20 to 100 rpm, preferably 45 to 70 rpm. The pellets are then molded in either a single material molding or multi-material molding machine, which maybe single cavity or multicavity, optionally equipped with a hot-runner system. The process temperature can be from 165°C to 250°C, preferably from 180°C to 225 °C. The injection pressure should be sufficient to fill the part completely without flashing. Depending on the cavity size, configuration and quantity, the injection pressure can range from 300 to 2500 psi (2.07 - 17.2 MPa). The cycle time is dependent on the same parameters and can range from 3 to 30 seconds, with the optimum generally being about 6 to 15 seconds.

### Example 2:

The lubricating members were prepared by dry mixing the dry ingredients in the table above and then spray coating the silwet L7210 onto the powder blend. The blended components may be extruded through a Rondo 118, 18mm diameter extruder with a barrel pressure of about 500-1000 psi (∼3.44 - 6.89 MPa), a rotor speed of about 10 to 50 rpm, and a temperature of about 100°-160° C and a die temperature of about 100°C-160°C. Alternatively, a 1½ inch (∼3.81 cm) single screw extruder may be employed with a processing temperature of 100°C-160°C, preferably 110-130°C, a screw speed of 20 to 50 rpm, preferably 25 to 35 rpm, and an extrusion pressure of 1800 to 7500 psi (∼12.4 - 51.7 MPa), preferably 4000 to 6500 psi (∼27.6 - 44.8 MPa). The extruded strip is cooled to about 25°C. To injection mold the strips it is preferred to first extrude the powder blend into pellets. This can be done on a 1¼ or 1½ inch (∼3.18 - 3.81 cm) single screw extruder at a temperature of 100°-140° C, preferably 110°- 130°C, with a screw speed of 20 to 100 rpm, preferably 45 to 70 rpm. The pellets are then molded in either a single material molding or multi-material molding machine, which may be single cavity or multi-cavity, optionally equipped with a hot-runner system. The process temperature can be from 100°C to 185°C, preferably from 110°C to 145°C. The injection pressure should be sufficient to fill the part completely without flashing. Depending on the cavity size, configuration and quantity, the injection pressure can range from 300 to 2500 psi (∼2.07 - 17.2 MPa). The cycle time is dependent on the same parameters and can range from 3 to 30 seconds, with the optimum generally being about 6 to 15 seconds. In one embodiment, one or more feeds can be preheated or they can be fed in at ambient temperature.

### Example 3

The lubricating members were made according to example 1 except that Silwet is added after formation of the lubricating member. The lubricating members are loaded into a low shear paddle mixer with the mixer running at sufficient speed to create a fluidized zone, Silwet was introduced into the mixing zone over 20 seconds, with continued mixing for an additional 10 seconds after Silwet addition was complete. Alternatively, the lubricating member may be coated continuously via direct application (immersion, spray) or transfer from an applicator (roller, pad, etc.) to the surface of the member during extrusion and/or at product assembly. Asusedherein, molecular weights (mol. wt.s) are provided in unified atomic mass units, daltons, or g/mol.

All parts, ratios, and percentages herein, in the Description, Examples, and Claims, are by weight of the lubricating member and all numerical limits are used with the normal degree of accuracy afforded by the art, unless otherwise specified.

The dimensions and values disclosed herein are not to be understood as being strictly limited to the exact numerical values recited. Instead, unless otherwise specified, each such dimension is intended to mean both the recited value and a functionally equivalent range surrounding that value.

## Claims

1. A lubricating member for use on a hair removal device, said lubricating member comprising a lubricating material comprising a) from 1 % to 99% by weight of a water soluble polymer and b) from 0.1% to 70% by weight of a silicone polyether block copolymer wherein said silicone polyether block copolymer comprises from 1 to 50%, by weight of polyethylene oxide, from 20% to 90% by weight of polypropylene oxide and from 1 to 20%, more preferably 15% by weight of silicone, wherein said silicone polyether block copolymer excludes additional alkyl chains.

2. A lubricating member according to claim 1, wherein said silicone polyether block copolymer comprises from 1 % to 30% by weight of polyethylene oxide, from 20% to 80% by weight of polypropylene oxide and from 1 % to 20% by weight of silicone.

3. The lubricating member according to any one of the proceeding claims, wherein said silicone polyether block copolymer has a molecular weight of from 10000 to 19000, preferably from 10000 to 15000.

4. The lubricating member according to any one of the preceding claims, wherein said silicone polyether block copolymer has a ratio of polyethylene oxide to polypropylene oxide of from 2.0 to 0.1, more preferably from 0.6 to 0.25.

5. The lubricating member according to claim 1, wherein the ratio of said water soluble polymer to said silicone block copolymer is from 1:8 to 8:1, preferably from 1:5 to 5:1, more preferably from 3:1 to 1:3.

6. The lubricating member according to any one of the preceding claims wherein said water soluble polymer is selected from polyethylene oxide, polyvinyl pyrrolidone, polyacrylamide, polyhydroxymethacrylate, polyvinyl imidazoline, polyethylene glycol, polyvinyl alcohol, polyhydroxyethymethacrylate, guars, cellulose, modified cellulose and mixtures thereof.

7. The lubricating member according to any one of the preceding claims, wherein said water soluble polymer is polyethylene oxide having an average molecular weight of at least 300000, preferably from 300,000 to 8 Million, more preferably from 1 million to 5 million, most preferably from 2 to 3 million.

8. The lubricating member according to any one of the preceding claims, comprising from 15% to 60% by weight of said water soluble polymer and from 1% to 20% by weight of said silicone polyether block copolymer.

9. The lubricating member according to claim 6, wherein said water soluble polymer further comprises from 0.01% to 50%, preferably from 2% to 40%, by weight of the lubricating material of a copolymer of polyethylene oxide and polypropylene oxide.

10. The lubricating member according to claim 6, wherein said polyethylene oxide polymer is present at a level of from 15% to 70%, preferably from 20% to 60%, more preferably from 25% to 50% by weight of the lubricating material.

11. The lubricating member according to any one of the preceding claims, wherein said lubricating member further comprises from 1% to 50% by weight of a water insoluble material, preferably selected from polyethylene, polypropylene, polystyrene, high impact polystyrene, butadiene styrene copolymer, polyacetal, acrylonitrile-butadiene styrene copolymer, ethylene vinyl acetate copolymer and mixtures thereof.

12. The lubricating member according to any one of the preceding claims, wherein said lubricating member has a coefficient of friction of 0.0300 or less, preferably of 0.0275 or less.

13. A hair removal cartridge having a front end and an opposing rear end, the hair removal cartridge comprising:
a. at least one hair removal member positioned between said front end and said rear end; and
b. at least one lubricating member according to any one of the preceding claims.

14. A hair removal device comprising:
a. a hair removal cartridge according to claim 13, and
b. a handle permanently or removably attached to said hair removal cartridge.

## Patentansprüche

1. Gleitelement zur Verwendung an einer Haarentfernungsvorrichtung, wobei das Gleitelement ein Gleitmaterial umfasst, das Folgendes umfasst: a) von 1 Gew.-% bis 99 Gew.-% ein wasserlösliches Polymer und b) von 0,1 Gew.-% bis 70 Gew.-% ein Silikonpolyetherblockcopolymer, wobei das Silikonpolyetherblockcopolymer von 1 bis 50 Gew.-% Polyethylenoxid, von 20 Gew.-% bis 90 Gew.-% Polypropylenoxid und von 1 bis 20 Gew.-%, besonders bevorzugt 15 Gew.-% Silikon, umfasst, wobei das Silikonpolyetherblockcopolymer zusätzliche Alkylketten ausschließt.

2. Gleitelement nach Anspruch 1, wobei das Silikonpolyetherblockcopolymer von 1 bis 30 Gew.-% Polyethylenoxid, von 20 Gew.-% bis 80 Gew.-% Polypropylenoxid und von 1 Gew-% bis 20 Gew.-% Silikon umfasst.

3. Gleitelement nach einem der vorstehenden Ansprüche, wobei das Silikonpolyetherblockcopolymer ein Molekulargewicht von 10000 bis 19000, vorzugsweise von 10000 bis 15000, aufweist.

4. Gleitelement nach einem der vorstehenden Ansprüche, wobei das Silikonpolyetherblockcopolymer ein Verhältnis von Polyethylenoxid zu Polypropylenoxid von 2,0 bis 0,1, besonders bevorzugt von 0,6 bis 0,25, aufweist.

5. Gleitelement nach Anspruch 1, wobei das Verhältnis des wasserlöslichen Polymers zu dem Silikonblockcopolymer von 1:8 bis 8:1, bevorzugt von 1:5 bis 5:1, besonders bevorzugt von 3:1 bis 1:3.

6. Gleitelement nach mindestens einem der vorstehenden Ansprüche, wobei das wasserlösliche Polymer ausgewählt ist aus Polyethylenoxid, Polyvinylpyrrolidon, Polyacrylamid, Polyhydroxymethacrylat, Polyvinylimidazolin, Polyethylenglykol, Polyvinylalkohol, Polyhydroxyethymethacrylat, Guar, Cellulose, modifizierter Cellulose und Mischungen davon.

7. Gleitelement nach einem der vorstehenden Ansprüche, wobei das wasserlösliche Polymer Polyethylenoxid mit einem mittleren Molekulargewicht von mindestens 300000, vorzugsweise von 300.000 bis 8 Millionen, besonders bevorzugt, 1 Millionen bis 5 Millionen, am meisten bevorzugt von 2 bis 3 Millionen, ist.

8. Gleitelement nach einem der vorstehenden Ansprüche, umfassend von 15 Gew.-% bis 60 Gew.-% des wasserlöslichen Polymers und von 1 Gew.-% bis 20 Gew.-% des Silikonpolyetherblockcopolymers.

9. Gleitelement nach Anspruch 6, wobei das wasserlösliche Polymer ferner von 0,01 Gew.-% bis 50 Gew.-%, vorzugsweise von 2 Gew.-% bis 40 Gew,-% des Gleitmaterial eines Copolymers von Polyethylenoxid und Polypropylenoxid umfasst.

10. Gleitelement nach Anspruch 6, wobei das Polyethylenoxid-Polymer mit einem Niveau von 15 Gew.-% bis 70 Gew.-%, vorzugsweise von 20 Gew.-% bis 60 Gew.-%, besonders bevorzugt von 25 Gew.-% bis 50 Gew,-% des Gleitmaterials vorhanden ist.

11. Gleitelement nach einem der vorstehenden Ansprüche, wobei das Gleitelement ferner von 1 Gew.-% bis 50 Gew.-% eines wasserunlöslichen Materials umfasst, vorzugsweise ausgewählt aus Polyethylen, Polypropylen, Polystyrol, hochschlagzähem Polystyrol, Butadien-Styrol-Copolymer, Polyacetal, Acrylnitril-Butadien-Copolymer, Ethylen-Vinylacetat-Copolymer und Mischungen davon, umfasst.

12. Gleitelement nach einem der vorstehenden Ansprüche, wobei das Gleitelement einen Reibungskoeffizienten von 0,0300 oder weniger, vorzugsweise von 0,0275 oder weniger, aufweist.

13. Haarentfernungskartusche mit einem vorderen Ende und einem dazu entgegengesetzten hinteren Ende, wobei die Haarentfernungskartusche Folgendes umfasst:
a. mindestens ein Haarentfernungselement, das zwischen dem vorderen Ende und dem hinteren Ende positioniert ist; und
b. mindestens ein Gleitelement nach einem der vorstehenden Ansprüche.

14. Haarentfernungsvorrichtung, umfassend:
a. eine Haarentfernungskartusche nach Anspruch 13, und
b. einen Griff, der permanent oder entfernbar an der Haarentfernungskartusche befestigt ist.

## Revendications

1. Élément de lubrification pour utilisation sur un dispositif d'épilation, ledit élément de lubrification comprenant un matériau lubrifiant comprenant a) de 1 % à 99 % en poids d'un polymère hydrosoluble et b) de 0,1 % à 70 % en poids d'un copolymère séquencé de polyéther de silicone dans lequel ledit copolymère séquencé de polyéther de silicone comprend de 1 à 50 %, en poids d'oxyde de polyéthylène, de 20 % à 90 % en poids d'oxyde de polypropylène et de 1 à 20 %, plus préférablement 15 % en poids de silicone, dans lequel ledit copolymère séquencé de polyéther de silicone exclut des chaînes alkyle supplémentaires.

2. Élément de lubrification selon la revendication 1, dans lequel ledit copolymère séquencé de polyéther de silicone comprend de 1 % à 30 % en poids d'oxyde de polyéthylène, de 20 % à 80 % en poids d'oxyde de polypropylène et de 1 % à 20 % en poids de silicone.

3. Élément de lubrification selon l'une quelconque des revendications précédentes, dans lequel ledit copolymère séquencé de polyéther de silicone a une masse moléculaire allant de 10 000 à 19 000, de préférence de 10 000 à 15 000.

4. Élément de lubrification selon l'une quelconque des revendications précédentes, dans lequel ledit copolymère séquencé de polyéther de silicone a un rapport de l'oxyde de polyéthylène à l'oxyde de polypropylène allant de 2,0 à 0,1, plus préférablement de 0,6 à 0,25.

5. Élément de lubrification selon la revendication 1, dans lequel le rapport dudit polymère hydrosoluble audit copolymère séquencé de silicone va de 1:8 à 8:1, de préférence de 1:5 à 5:1, plus préférablement de 3:1 à 1:3.

6. Élément de lubrification selon l'une quelconque des revendications précédentes dans lequel ledit polymère hydrosoluble est choisi parmi oxyde de polyéthylène, polyvinylpyrrolidone, polyacrylamide, polyhydroxyméthacrylate, polyvinylimidazoline, polyéthylène glycol, alcool polyvinylique, polyhydroxyéthyméthacrylate, gommes de guar, cellulose, cellulose modifiée et des mélanges de ceux-ci.

7. Élément de lubrification selon l'une quelconque des revendications précédentes, dans lequel ledit polymère hydrosoluble est de l'oxyde de polyéthylène ayant une masse moléculaire moyenne d'au moins 300 000, de préférence de 300 000 à 8 millions, plus préférablement de 1 million à 5 millions, le plus préférablement de 2 à 3 millions.

8. Élément de lubrification selon l'une quelconque des revendications précédentes, comprenant de 15 % à 60 % en poids dudit polymère hydrosoluble et de 1 % à 20 % en poids dudit copolymère séquencé de polyéther de silicone.

9. Élément de lubrification selon la revendication 6, dans lequel ledit polymère hydrosoluble comprend en outre de 0,01 % à 50 %, de préférence de 2 % à 40 %, en poids du matériau lubrifiant d'un copolymère d'oxyde de polyéthylène et d'oxyde de polypropylène.

10. Élément de lubrification selon la revendication 6, dans lequel ledit polymère d'oxyde de polyéthylène est présent à un taux allant de 15 % à 70 %, de préférence de 20 % à 60 %, plus préférablement de 25 % à 50 % en poids du matériau lubrifiant.

11. Élément de lubrification selon l'une quelconque des revendications précédentes, dans lequel ledit élément de lubrification comprend en outre de 1 % à 50 % en poids d'un matériau insoluble dans l'eau, choisi de préférence parmi polyéthylène, polypropylène, polystyrène, polystyrène à haut impact, copolymère butadiène-styrène, polyacétal, copolymère acrylonitrile-butadiène-styrène, copolymère éthylène-acétate de vinyle et des mélanges de ceux-ci.

12. Élément de lubrification selon l'une quelconque des revendications précédentes, dans lequel ledit élément de lubrification a un coefficient de frottement de 0,0300 ou moins, de préférence de 0,0275 ou moins.

13. Cartouche d'épilation ayant une extrémité avant et une extrémité arrière opposée, la cartouche d'épilation comprenant :
a. au moins un élément d'épilation positionné entre ladite extrémité avant et ladite extrémité arrière ; et
b. au moins un élément de lubrification selon l'une quelconque des revendications précédentes.

14. Dispositif d'épilation comprenant :
a. une cartouche d'épilation selon la revendication 13, et
b. une poignée fixée de façon permanente ou amovible à ladite cartouche d'épilation.
